Europäisches Patentamt

(19) European Patent Office

Office européen des brevets

(11) Publication number: **0 259 988**

**A2**

## (12) EUROPEAN PATENT APPLICATION

(21) Application number: 87307240.9

(51) Int. Cl.⁴: **A61M 5/00**

(22) Date of filing: 17.08.87

(30) Priority: 12.09.86 GB 8622054

(43) Date of publication of application:
16.03.88 Bulletin 88/11

(84) Designated Contracting States:
DE ES FR IT NL SE

(71) Applicant: The BOC Group plc
Chertsey Road
Windlesham Surrey GU20 6HJ(GB)

(72) Inventor: Dickens, Robert Andrew
4 Salop Close Shrivenham
Nr Swindon Wiltshire(GB)
Inventor: Scott, Ian Laurence
11 Sterling Close Wroughton
Swindon Wiltshire(GB)
Inventor: McBride, Archibald
26 Ball Road
Pewsey Wiltshire(GB)

(74) Representative: Gough, Peter
c/o THE BOC GROUP PLC Patent Department
Chertsey Road
Windlesham Surrey GU20 6HJ(GB)

(54) Improvements in or relating to cannula assemblies.

(57) A cannula assembly comprises a connection housing 2 having a through passageway 4 for the passage therethrough of a puncture needle 22 and a side limb 8 in communication with the passageway 4. The side limb 8 accommodates a pressure sensor 10. A flexible cannula tube 1 for insertion into a patient is connected to one end of the passageway 4.

FIG. 2.

EP 0 259 988 A2

## IMPROVEMENTS IN OR RELATING TO CANNULA ASSEMBLIES

The present invention relates to cannula assemblies and more particularly to cannula assemblies which incorporate a blood pressure sensor and are suitable for use with the over needle technique of insertion into the body of a patient.

It is known for an infusion cannula assembly of the type suitable for over needle insertion into a patient to include an elongate connection housing having a through passageway extending from one end of the housing to the other in the longitudinal direction of the housing. A cannula tube of flexible material is attached to the housing and extends from one end of the through passageway. The opposite end of the passageway is designed as a holder sleeve for the releasable accommodation of a coupling piece. A puncture needle unit forms part of the cannula assembly and has a head releasably accommodated in the holder sleeve of the connection housing. A puncture needle of metal projects from the head. The needle is pointed at its free end distal from the head and extends from the head through the passageway of the connection housing and on through the flexible cannula tube and projects slightly with its pointed end out from the distal end of the flexible cannula tube.

In use when employing the over needle technique, the spiculated end of the metal needle enters a patient's blood vessel and this will be followed by the flexible cannula tube. Once full penetration of the patient's blood vessel by the cannula tube has been achieved, the puncture needle is released from the connection housing and the needle withdrawn through the cannula tube leaving the cannula tube in situ connected to the connection housing.

If it becomes necessary to measure the blood pressure of the patient then the cannula tube and connection housing are filled with a fluid and coupled by tubing to an external pressure transducer so that the blood pressure is transmitted hydraulically to the pressure transducer.

This type of apparatus has certain disadvantages, for example, all the extra components between the cannula and the transducer reduce the fidelity of the pressure signal. Further each component increases the risk of air entrapment. The presence of air significantly reduces the fidelity of the pressure signal and can represent a hazard to patient safety if infused in the patients blood stream. As a result, setting up the apparatus is extremely time consuming as all traces of air must be meticulously flushed out.

It is known, for example, from United States Patent No. 3 215 135 for a blood pressure measuring device to include means in the form of a housing for supporting a light reflecting diaphragm deformable in response to the pressure of a fluid applied directly to one face of the diaphragm. Two flexible light guides extend into the housing and terminate at a location spaced from and adjacent the diaphragm. In use, one light guide conducts light from a source and emits it on to the opposite face of the diaphragm which reflects a proportional part of the light from the emitter towards a receiver light guide.

A disadvantage of this blood pressure monitoring device is that it cannot be inserted with the overneedle technique. The device must be introduced following cannulation of the vessel. The small size of the device required for intravascular use means that its manufacture is extremely difficult. The calibration of an intravascular device in-situ is an additional difficulty.

It is an aim of the present invention to provide a cannula assembly which can be used with the over needle technique and has an inbuilt blood pressure measuring facility.

It is a further aim of the present invention to provide a cannula assembly which overcomes both the disadvantages of remote pressure monitoring and the problems of using an intravascular sensor.

According to the present invention, a cannula assembly comprises a connection housing having a through passageway, a cannula tube of flexible material for insertion into a patient connected to one end of the passageway and extending out from the connection housing and a pressure sensor mounted on the connection housing for sensing the pressure of fluid in the passageway.

Preferably, the pressure sensor is located in a side limb of the connection housing in communication with the passageway.

An embodiment of the invention will now be described by way of example, reference being made to the Figures of the accompanying diagrammatic drawings in which:-

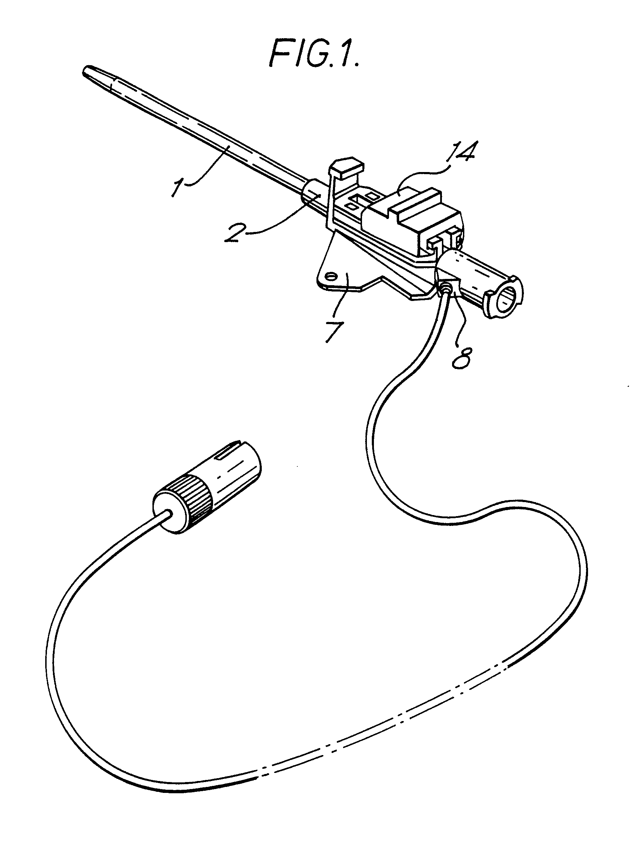

Figure 1 is a perspective view of part of a cannula assembly incorporating a pressure sensor according to the present invention;

Figure 2 is a plan view partly in cross-section of the cannula assembly of Figure 1 together with a puncture needle unit; and

Figure 3 is an enlarged detail view of part of the cannula assembly illustrated in Figure 2.

As shown, a cannula assembly includes a cannula tube 1 made from an elastic flexible material one end of which tube is mounted in a tubular connection housing 2. The connection housing 2 is made of relatively rigid material, for example, polyethelene and has a through passageway 4 to which the tube 1 is connected in a manner known per se. At its right-hand (as shown) end, the wall of the housing 2 is tapered to form a connecting sleeve 6. Wings 7 project from the base of the connection housing 2 which wings facilitate strapping the cannula assembly to a patient.

A side limb 8, is formed in the wall of the connection housing 2 between the opposed ends of the housing 2. A through passage is formed in the side limb 8 which passage accommodates a pressure sensor 10.

Within the passageway 4 is a resilient tubular hose 12 which can be squeezed to a closed position to prevent the passage of fluid therethrough. The resilient hose 12 is squeezed by means of a cam actuated ball (not shown) operated by a two position switch 14 mounted on the connection having 2 in a manner per se. The switch 14 can be used to calibrate the sensor 10 as will be explained.

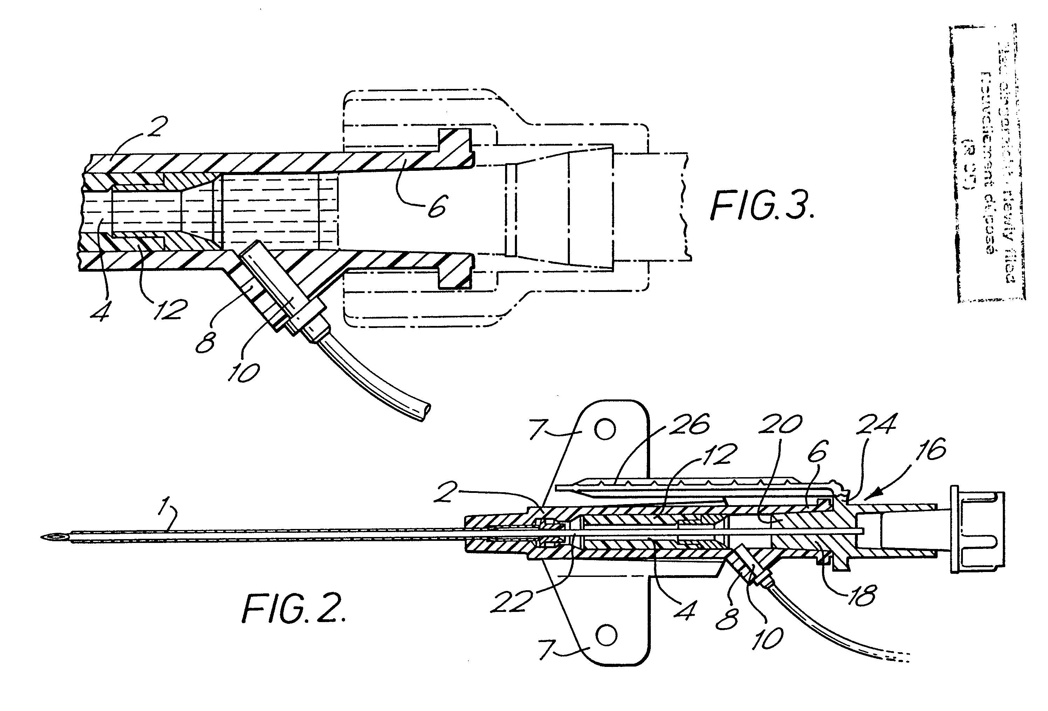

Referring in particular to Figure 2 a puncture needle unit 16 includes a head 18 with a conical portion 20 which is releasably received in the sleeve 6. A metal needle 22 projects from the head 18 and extends through the passageway 4, hose 12 and through the cannula tube 1 as shown. The metal needle 22 projects slightly with its pointed end out from the distal end of the flexible cannula tube 1.

A circumferential flange 24 is provided integral with the head 18 from which extend, parallel with the housing 2, a pair of opposed finger grip wings 26 (only one shown).

The pressure sensor 10 may be of the type as described in our co-pending UK patent application No. 8618845 (BOC Reference 8621).

When using the over needle technique, the metal needle 22 is inserted into the blood vessel of a patient and the flexible cannula tube 1 will automatically follow and enter the blood vessel a predetermined distance. The puncture needle unit 16 is then detached from the connecting housing 2 and withdrawn from the blood vessel, the metal needle 22 passing through the cannula tube 1 and the passageway 4 of the connecting housing 2. Thus the cannula tube 1 remains in the blood vessel of the patient and the pressure of the back flow of blood through the cannula tube and into the cavity 4 will be measured at the pressure sensor 10.

The sensor 10 is usually calibrated prior to use and the calibration is checked during use by exposing the sensor 10 to a reference pressure source. With the switch 14 in its closed position, that is, with the resilient hose 12 squeezed the sensor 10 can be exposed to any reference pressure connected to a female luer. Once calibrated, the female luer is plugged and the switch 14 moved to its open position to allow the sensor 10 to be exposed to blood pressure via the cannula tube 1.

It will be apparent, that with the cannula assembly just described the over needle insertion technique can be used but the disadvantages of using an external pressure transducer are avoided. Since the pressure is measured almost at source the fidelity of the signal is high and there is less chance of air entrainment. Further the elimination of the conventional hydraulic line provides a substantial saving in setting up time. There is also a saving in the cost of components and nursing time.

## Claims

1. A cannula assembly comprising a connection housing (2) having a through passageway (4), a cannula tube (1) of flexible material for insertion into a patient connected to one end of the passageway (4) and extending out from the connection housing (2), characterised by a pressure sensor (10) mounted on the connection housing (2) for sensing the pressure of fluid in the passageway (4).

2. A cannula assembly as claimed in claim 1, characterised in that the pressure sensor (10) is located in a side limb (8) of the connection housing (2) in communication with the passageway (4).

3. A cannula assembly as claimed in claim 1 or 2, characterised in that the passageway (4) accommodates a resilient tubular hose (12) and means (14) for squeezing the hose (12) to a closed position to prevent the flow of fluid through the passageway (4).

4. A cannula assembly as claimed in claim 3, characterised in that a puncture needle (22) extends through the passageway (4), the hose (12) and the cannula tube (1) and projects slightly with its pointed end out from the distal end of the cannula tube (1).

0 259 988

# FIG.1.

FIG.3.

FIG.2.

0 259 988